# EUROPEAN PATENT SPECIFICATION

(11) **EP 3 351 933 B1**
(45) Date of publication and mention of the grant of the patent: **21.08.2019**
(21) Application number: 17210196.6
(22) Date of filing: 22.12.2017
(51) Int. Cl.: G01N 27/417, G01N 33/00

(54) **DIAGNOSIS APPARATUS FOR EXHAUST GAS SENSOR**
DIAGNOSEVORRICHTUNG FÜR EINEN ABGASSENSOR
APPAREIL DE DIAGNOSTIC POUR CAPTEUR DE GAZ D'ÉCHAPPEMENT

(30) Priority: 26.12.2016 JP 2016251520
(43) Date of publication of application: 25.07.2018
(73) Proprietor: TOYOTA JIDOSHA KABUSHIKI KAISHA, Toyota-shi, Aichi, 471-8571 (JP)
(72) Inventor: MIYAMOTO, Hiroshi, Toyota-shi, Aichi 471-8571 (JP); HAGIWARA, Koji, Toyota-shi, Aichi 471-8571 (JP); MASUBUCHI, Masahiko, Toyota-shi, Aichi 471-8571 (JP); IWAZAKI, Yasushi, Toyota-shi, Aichi 471-8571 (JP); KIDOKORO, Toru, Toyota-shi, Aichi 471-8571 (JP)
(74) Representative: D Young & Co LLP

(56) References cited:
- US-A1- 2005 016 253
- US-A1- 2016 069 242
- US-A1- 2016 123 922

## Description

### Technical Field

The present invention relates to a diagnosis apparatus for an exhaust gas sensor.

### Description of the Related Art

An air-fuel ratio sensor is provided in the exhaust system of some internal combustion engines to control the air-fuel ratio of the internal combustion engine. A NOx sensor is provided in the exhaust system of some internal combustion engines to control the quantity of reducing agent to be supplied to an exhaust gas purification apparatus including a selective catalytic reduction NOx catalyst.

A limiting current air-fuel ratio sensor is a known air-fuel ratio sensor. A limiting current NOx sensor is a known NOx sensor. The limiting current air-fuel ratio sensor and the limiting current NOx sensor have a measuring part that is configured to measure the oxygen concentration in the exhaust gas and a protective cover for protecting the measuring part. The measuring part comprises a pair of electrodes, oxygen ion conductive solid electrolyte sandwiched between the electrodes, and a porous diffusion rate limiting layer that allows the exhaust gas to travel to the exhaust gas side electrode among the two electrodes while limiting the rate of diffusion of the exhaust gas. The protective cover has a plurality of air holes.

In the limiting current air-fuel ratio sensor and the limiting current NOx sensor, when the exhaust gas having passed through the diffusion rate limiting layer reaches the exhaust gas side electrode while a voltage is applied between the electrodes, an electrical current flows between the electrodes by propagation of oxygen ions between the electrodes through the oxygen ion conductive solid electrolyte. Since the diffusion of the exhaust gas toward the exhaust gas side electrode is limited by the diffusion rate limiting layer, there is a range of applied voltage in which the current is saturated at a constant value even if the applied voltage is increased. The air-fuel ratio sensor and the NOx sensor can determine the air-fuel ratio and the NOx concentration in the exhaust gas respectively by measuring this current value, which is called the limiting current value.

US 2016/069242 A1 discloses an abnormality diagnosis system of an air-fuel ratio sensor.

### Citation List

### Patent Literature

Patent Literature 1: Japanese Patent Application Laid-Open No. 2004-003513
Patent Literature 2: Japanese Patent Application Laid-Open No. 2015-175306
Patent Literature 3: Japanese Patent Application Laid-Open No. 2010-007534
Patent Literature 4: Japanese Patent Application Laid-Open No. 2005-121003

### SUMMARY OF THE INVENTION

### Technical problem

It is conventionally known to determine deterioration of an air-fuel ratio sensor on the basis of the rate of change of the output of the air-fuel ratio sensor. In this conventional method, the change rate of the sensor output is calculated as the air-fuel ratio is increased by performing fuel cut. Deteriorations of the air-fuel ratio sensor include elongation of the time taken for the sensor output to converge to a constant value (i.e. deterioration in its responsiveness) and a decrease in the limiting current value. The change rate of the output refers to the change of the output per unit time. When the change rate defined as above is lower than a threshold, it is determined that the responsiveness of the air-fuel ratio sensor is deteriorated.

The change rate of the output of air-fuel ratio sensors sometimes decreases even though the responsiveness thereof is not deteriorated. This is the case, for example, in situations where the limiting current value is decreased while the time taken for the sensor output to converge is normal. Since there may be such situations, if a diagnosis as to the responsiveness of an air-fuel ratio sensor is made only on the basis of the change rate of the sensor output, there is a possibility that abnormality of the responsiveness of the sensor cannot be determined correctly. Moreover, if the responsiveness of an air fuel ratio sensor is estimated only on the basis of the change rate of the sensor output, the accuracy of the estimation may be deteriorated. This is the case not only with air-fuel ratio sensors but also with NOx sensors.

The present invention has been made to solve the above problem, and an object of the present invention is to estimate the responsiveness of an exhaust gas sensor with as high accuracy as possible and to determine abnormality in the responsiveness of an exhaust gas sensor accurately.

### Solution to Problem

The present invention relates to a diagnosis apparatus for an exhaust gas sensor applied to an internal combustion engine. The exhaust gas sensor has a measuring part that is configured to measure the oxygen concentration in the exhaust gas comprising a pair of electrodes including an exhaust gas side electrode exposed to the exhaust gas and an atmosphere side electrode exposed to the atmosphere and a porous diffusion rate limiting layer that allows the exhaust gas to travel to the exhaust gas side electrode while limiting the rate of diffusion of the exhaust gas delivered to the exhaust gas side electrode, and a protective cover that is configured to enclose said measuring part and including a plurality of air holes. The exhaust gas sensor outputs an output signal representing the oxygen concentration in the exhaust gas.

This exhaust gas sensor is what is called a limiting current exhaust gas sensor. In this exhaust gas sensor, since the rate of diffusion of exhaust gas to the exhaust gas side electrode is limited by the diffusion rate limiting layer, there is a range of applied voltage in which the electrical current is saturated at a constant value even if the applied voltage is increased. The air-fuel ratio of the exhaust gas or the NOx concentration in the exhaust gas can be determined by measuring this value of the electrical current (or the limiting current value).

The responsiveness and the limiting current value of the exhaust gas sensor according to the present invention can change due to various factors such as manufacturing variations, deteriorations of the diffusion rate limiting layer, adhesion of particulate matter and/or oil components to the protective cover, differences in the environment in which the exhaust gas sensor is used. These factors will be hereinafter referred to as the "influential factors". Specifically, for example, smaller electrode areas due to manufacturing variations lead to smaller limiting current values as compared to larger electrode areas, and larger thicknesses of the diffusion rate limiting layer due to manufacturing variations lead to smaller limiting current values as compared to smaller thicknesses. Furthermore, the limiting current value is smaller when the pressure of the exhaust gas around the exhaust gas sensor is low than when it is high. Decreases in the limiting current value tend to lead to decrease in the absolute value of the change rate of the sensor output.

If particulate matter, oil components or the like adhere to the air holes of the protective cover, the quantity of exhaust gas flowing into the interior of the protective cover per unit time is apt to decrease. When the quantity of exhaust gas flowing into the interior of the protective cover per unit time decreases, if the concentrations of components such as O₂, HC, CO, and NOx in the exhaust gas discharged from the internal combustion engine change depending on the operation state of the internal combustion engine, it takes some time until the concentrations of these components in the exhaust gas inside the protective cover change to the concentrations corresponding to the operation state of the internal combustion engine (namely, the concentrations in the exhaust gas discharged from the internal combustion engine). The exhaust gas flowing into the interior of the protective cover travels through the diffusion rate limiting layer to reach the exhaust gas side electrode, and oxygen ions propagate between the pair of electrodes including the exhaust gas side electrode and the atmosphere side electrode, resulting in an electrical current flowing between the electrodes. Therefore, until the concentrations of components such as O₂, HC, CO, and NOx in the exhaust gas inside the protective cover change to the concentrations corresponding to the operation state of the internal combustion engine, the output of the exhaust gas sensor does not agree with the concentrations corresponding to the operation state of the internal combustion engine. This means that the responsiveness of the exhaust gas sensor is deteriorated. Deteriorations in the responsiveness tend to lead to decreases in the absolute value of the change rate of the sensor output.

If particulate matter, oil components or the like adhere to the porous diffusion rate limiting layer, the porosity of the diffusion rate limiting layer is apt to decrease. On the other hand, if the diffusion rate limiting layer is deteriorated, the porosity of the diffusion rate limiting layer is apt to increase. When the porosity of the diffusion rate limiting layer becomes lower, the resistance against the flow of exhaust gas through the diffusion rate limiting layer becomes higher than when the porosity is high. Hence, in the limiting current exhaust gas sensor, the lower the porosity of the diffusion rate limiting layer is, the smaller the current value at which the electrical current is saturated tends to be. In other words, the lower the porosity of the diffusion rate limiting layer is, the more the limiting current value is apt to decrease. Moreover, since the rate of diffusion of the exhaust gas delivered to the exhaust gas side electrode changes with changes in the porosity of the diffusion rate limiting layer, changes in the porosity of the diffusion rate limiting layer can lead to not only changes in the limiting current value but also changes in the responsiveness of the exhaust gas sensor. When the output of the exhaust gas sensor is affected by changes in the porosity of the diffusion rate limiting layer, the limiting current value is affected more seriously than the responsiveness of the exhaust gas sensor.

As described above, when particulate matter, oil components or the like adhere to the protective cover, if the concentrations of components such as O₂, HC, CO, and NOx in the exhaust gas discharged from the internal combustion engine change depending on the operation state of the internal combustion engine, it may take a relatively long time for the output of the exhaust gas sensor to converge to the output that corresponds to the concentrations of these components. In other words, there is a possibility that the responsiveness of the exhaust gas sensor may be deteriorated. In the exhaust gas sensor with deteriorated responsiveness, the absolute value of the change rate of the sensor output is smaller than that in the normal exhaust gas sensor. Therefore, it may be considered that the responsiveness of the exhaust gas sensor can be estimated on the basis of the change rate of the sensor output.

However, the output of the above-described exhaust gas sensor is combinedly affected by the change in the responsiveness of the exhaust gas sensor (i.e. the change in the time taken for the output of the exhaust gas sensor to converge) and the change in the limiting current value. Therefore, if the responsiveness of the exhaust gas sensor is estimated only on the basis of the change rate of the sensor output, the accuracy of the estimation can be deteriorated. This is because in an exhaust gas sensor in which the limiting current value is decreased (or the limiting current value is abnormal), the absolute value of the change rate of the sensor output may decrease despite that the responsiveness of the sensor is not deteriorated.

A diagnosis apparatus for an exhaust gas sensor according to a first aspect of the present invention comprises a control unit that is configured to perform oxygen concentration control to change the oxygen concentration in the exhaust gas, a calculation unit that is configured to calculate a responsiveness index correlating with the responsiveness of said exhaust gas sensor by dividing the largest value of an absolute change rate defined as the absolute value of the change rate of said output signal during a reference period defined as a period from the time at which said oxygen concentration control starts to be performed to the time at which said output signal changing with the performance of said oxygen concentration control converges by the difference between the value of said output signal at the time when said absolute change rate becomes largest and the converged value of said output signal with said reference period, and a responsiveness diagnosis unit that is configured to estimate the responsiveness of said exhaust gas sensor on the basis of said responsiveness index.

When the oxygen concentration control starts to be performed in the above-described diagnosis apparatus for an exhaust gas sensor, the oxygen concentration in the exhaust gas starts to change, and the oxygen concentration in the exhaust gas that reaches the exhaust gas side electrode of the exhaust gas sensor starts to change accordingly. In consequence, the value of the output current of the exhaust gas sensor (that is, in this case, the limiting current value) changes. After the end of the oxygen concentration control, the oxygen concentration in the exhaust gas converges to a specific concentration, and the oxygen concentration in the exhaust gas that reaches the exhaust gas side electrode of the exhaust gas sensor converges to this specific concentration accordingly. In consequence, the value of the output current of the exhaust gas sensor (i.e. the limiting current value) converges to a value corresponding to the oxygen concentration (i.e. the aforementioned specific concentration) in the exhaust gas after the performance of the oxygen concentration control. As above, when the oxygen concentration control is performed, the output signal of the exhaust gas sensor changes accordingly.

In the oxygen concentration control, the oxygen concentration in the exhaust gas can be changed by changing the air-fuel ratio. For example, when the operation of the internal combustion engine shifts from normal operation to fuel cut, the air-fuel ratio changes to air-fuel ratios leaner than the air-fuel ratio before the shift to fuel cut (namely, the air-fuel ratio during the normal operation). Similarly, when the operation of the internal combustion engine shifts from fuel cut to normal operation, the air-fuel ratio changes to air-fuel ratios richer than the air-fuel ratio before the shift to normal operation (namely, the air-fuel ratio during fuel cut). In the oxygen concentration control, the oxygen concentration in the exhaust gas can be changed by changing the air-fuel ratio in this way. The method of changing the air-fuel ratio is not limited to the above-described method, but other known methods may be employed. For example, an air-fuel ratio before the oxygen concentration control (first air-fuel ratio) and an air-fuel ratio after the oxygen concentration control (second air-fuel ratio) may be set beforehand, and the air-fuel ratio may be changed from the first air-fuel ratio to the second air-fuel ratio by the oxygen concentration control.

When the oxygen concentration in the exhaust gas is changed by changing the air-fuel ratio, while the output signal of the exhaust gas sensor changes according to changes in the air-fuel ratio of the exhaust gas, changes of the output signal of the exhaust gas sensor are too small to measure in some cases until a certain length of time elapses since the start of the oxygen concentration control. After a certain length of time has elapsed since the start of the oxygen concentration control, changes of the output signal of the exhaust gas sensor become large enough to measure. The magnitude of the change rate of the output signal during the reference period, which is defined as a period from the time at which the oxygen concentration control starts to be performed to the time at which the output signal changing with the performance of the oxygen concentration control converges, tends to become largest after a certain length of time has elapsed since the start of the oxygen concentration control. Therefore, the responsiveness of the exhaust gas sensor can easily be assessed on the basis of the largest value of the absolute value of the change rate of the output signal during the aforementioned reference period. In the following, the absolute value of the change rate of the output signal will also be referred to as the "absolute change rate".

By calculating the responsiveness index by the calculation unit as above, influences of the aforementioned influential factors can be eliminated in cases where the limiting current value changes due to the influential factors. This is because the limiting current value corresponding to the converged oxygen concentration in the exhaust gas after the end of the oxygen concentration control and the converged value of the output signal with the aforementioned reference period are equal to each other, and therefore the influences in the case where the limiting current value changes are eliminated by dividing the largest value of the absolute change rate by the difference between the value of the output signal at the time when the absolute change rate becomes largest and the converged value of the output signal with the aforementioned reference period. The responsiveness diagnosis unit estimates the responsiveness of the exhaust gas sensor on the basis of the responsiveness index calculated in this way. The responsiveness diagnosis unit may estimate the responsiveness of the exhaust gas sensor higher when the responsiveness index is large than when it is small. Thus, the responsiveness of the exhaust gas sensor can be estimated with as high accuracy as possible.

Specifically, if the limiting current value is decreased due to an abnormality, the converged value of the output signal with the reference period is also decreased. If the largest value of the absolute change rate were simply used as a responsiveness index when the time taken for the output signal to converge does not change (i.e. the responsiveness of the exhaust gas sensor does not change) though the limiting current value is decreased, it would be erroneously assessed that the responsiveness is deteriorated with decreasing limiting current value, because the largest value of the absolute change rate decreases with decreasing limiting current value. In contrast, when the calculation unit calculates the responsiveness index in the above-described manner, influences in the case where the limiting current value changes are eliminated as described above, the responsiveness index hardly changes when the time taken for the output signal to converge does not change (in other words, the responsiveness of the exhaust gas sensor does not change) while the limiting current value changes. Therefore, an erroneous determination is prevented from being made.

As above, by calculating the responsiveness index in the above-described manner by the calculation unit, the responsiveness of the exhaust gas sensor can be assessed with improved accuracy. For example, even if the responsiveness of the exhaust gas sensor is deteriorated due to the adhesion of particulate matter, oil components or the like to the protective cover and the limiting current value is decreased due to the adhesion of particulate matter, oil components or the like to the diffusion rate limiting layer, the responsiveness of the exhaust gas sensor can be estimated with as high accuracy as possible by estimating the responsiveness of the exhaust gas sensor on the basis of the responsiveness index.

The diagnosis apparatus for an exhaust gas sensor according to the first aspect of the present invention estimates the responsiveness of the exhaust gas sensor on the basis of the above-described responsiveness index to thereby enable the estimation of the responsiveness of the exhaust gas sensor with as high accuracy as possible.

A diagnosis apparatus for an exhaust gas sensor according to the second aspect of the present invention comprises a control unit that is configured to perform oxygen concentration control to change the oxygen concentration in the exhaust gas, a calculation unit that is configured to calculate a responsiveness index correlating with the responsiveness of said exhaust gas sensor by dividing the largest value of an absolute change rate defined as the absolute value of the change rate of said output signal during a reference period defined as a period from the time at which said oxygen concentration control starts to be performed to the time at which said output signal changing with the performance of said oxygen concentration control converges by the difference between the value of said output signal at the time when said absolute change rate becomes largest and the converged value of said output signal with said reference period, and an abnormality diagnosis unit that is configured to diagnose abnormality in the responsiveness of said exhaust gas sensor on the basis of said responsiveness index.

The abnormality diagnosis unit may diagnose that the responsiveness of the exhaust gas sensor is abnormal for example when the responsiveness index is smaller than a predetermined threshold. The method of abnormality diagnosis is not limited to this. In any case, the abnormality diagnosis unit can determine whether or not there is an abnormality in the responsiveness of the exhaust gas sensor on the basis of the responsiveness index.

As described in the above description of the responsiveness index, the value of the responsiveness index hardly changes when the time taken for the output signal to converge does not change (in other words, the responsiveness of the exhaust gas sensor does not change) while the limiting current value changes. Therefore, the diagnosis of abnormality of the responsiveness of the exhaust gas sensor based on the responsiveness index can improve the accuracy of determination as to whether there is an abnormality in the responsiveness of the exhaust gas sensor.

The diagnosis apparatus for an exhaust gas sensor according to the second aspect of the present invention diagnoses abnormality in the responsiveness of the exhaust gas sensor on the basis of the responsiveness index as described above to thereby enable determination of an abnormality in the responsiveness of the exhaust gas sensor with improved accuracy.

### Advantageous Effects of Invention

The present invention makes it possible to estimate the responsiveness of an exhaust gas sensor with as high accuracy as possible and to determine abnormality in the responsiveness of an exhaust gas sensor accurately.

### BRIEF DESCRIPTION OF DRAWINGS

Fig. 1 is a diagram showing the general configuration of an internal combustion engine to which an air-fuel ratio sensor according to a first embodiment is applied and its air-intake and exhaust systems.
Fig. 2 is an enlarge cross sectional view schematically showing the portion around the air-fuel ratio sensor shown in Fig. 1.
Fig. 3 is a cross sectional view taken along line A-A' in Fig. 2.
Fig. 4 shows changes with time of a fuel cut execution flag, the output of the air-fuel ratio sensor, the sensor output change rate, and the highest output change rate, when the fuel cut process is performed.
Fig. 5 is a flow chart of a control flow executed by a diagnosis apparatus for an exhaust gas sensor according to an embodiment of the first aspect of the present invention (i.e. the first embodiment).
Fig. 6 shows changes with time of the fuel cut execution flag, the output of the air-fuel ratio sensor, the sensor output change rate, the highest output change rate, a first output value, a second output value, and a responsiveness index, when fuel cut is performed.
Fig. 7 is a flow chart of a control flow executed by a diagnosis apparatus for an exhaust gas sensor according to an embodiment of the second aspect of the present invention (a second embodiment).
Fig. 8 is a diagram showing the general configuration of an internal combustion engine to which an NOx sensor according to a third embodiment is applied and its air-intake and exhaust systems.
Fig. 9 is an enlarge cross sectional view schematically showing the portion around the NOx sensor shown in Fig. 8.
Fig. 10 is a cross sectional view taken along line B-B' in Fig. 9.

### DESCRIPTION OF EMBODIMENTS

In the following, modes for carrying out the present invention will be specifically described as embodiments for illustrative purposes with reference to the drawings. It should be understood that the dimensions, materials, shapes, relative arrangements, and other features of the components that will be described in connection with the embodiments are not intended to limit the technical scope of the present invention only to them, unless stated otherwise.

### < First Embodiment >

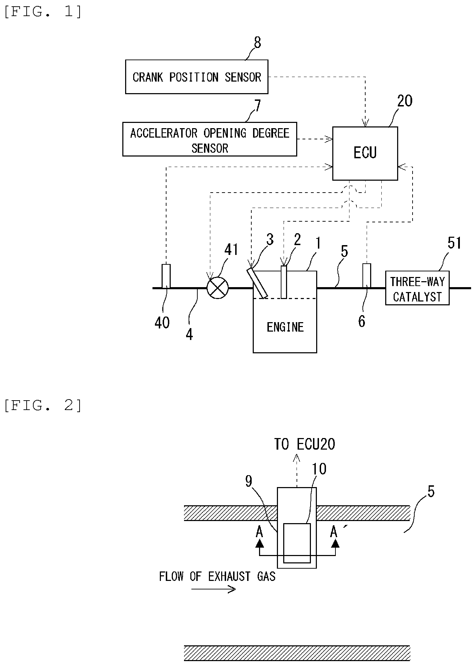

In the following, a first embodiment of the present invention will be described with reference to the drawings. Fig. 1 is a diagram showing the general configuration of an internal combustion engine according to an embodiment and its air-intake and exhaust systems. The internal combustion engine 1 shown in Fig. 1 is a spark-ignition internal combustion engine that uses as fuel gasoline or the like. The internal combustion engine has an ignition plug 2 and a fuel injection valve 3.

The internal combustion engine 1 is connected with an intake passage 4. The intake passage 4 is provided with an air flow meter 40 and a throttle valve 41. The air flow meter 40 outputs an electrical signal representing the quantity (mass) of intake air flowing in the intake passage 4. The throttle valve 41 is arranged in the intake passage 4 downstream of the air flow meter 40. The throttle valve 41 can vary the channel cross sectional area in the intake passage 4 to adjust the intake air quantity of the internal combustion engine 1.

The internal combustion engine 1 is connected with an exhaust passage 5. The exhaust passage 5 is provided with a three-way catalyst 51. The exhaust passage 5 is also provided with an air-fuel ratio sensor 6 arranged upstream of the three-way catalyst 51. The air-fuel ratio sensor 6 measures the air-fuel ratio of the exhaust gas flowing into the three-way catalyst 51. The air-fuel ratio sensor 6 will be specifically described later. The air-fuel ratio sensor 6 in this embodiment corresponds to the exhaust gas sensor according to the present invention.

An electronic control unit (ECU) 20 is provided for the internal combustion engine 1. The ECU 20 controls the operation state of the internal combustion engine 1. The ECU 20 is electrically connected with various sensors such as an accelerator opening degree sensor 7 and a crank position sensor 8 as well as the aforementioned air flow meter 40 and the air-fuel ratio sensor 6. The accelerator opening degree sensor 7 outputs an electrical signal representing the amount of operation of the accelerator pedal that is not shown in the drawings (or the accelerator opening degree). The crank position sensor 8 outputs an electrical signal representing the rotational position of the engine output shaft (or the crankshaft) of the internal combustion engine 1. The output signals of these sensors are input to the ECU 20. The ECU 20 calculates the engine load of the internal combustion engine 1 on the basis of the output signal of the accelerator opening degree sensor 7 and calculates the engine speed of the internal combustion engine 1 on the basis of the output signal of the crank position sensor 8. Moreover, the ECU 20 estimates the flow rate of the exhaust gas flowing into the three-way catalyst 51 on the basis of the output value of the air flow meter 40. The flow rate of the exhaust gas flowing into the three-way catalyst 51 will be also referred to as the "exhaust gas flow rate".

The ECU 20 is also electrically connected with various components including the ignition plug 2, the fuel injection valve 3, and the throttle valve 41. These components are controlled by the ECU 20. For example, the ECU 20 determines the air-fuel ratio of the exhaust gas flowing into the three-way catalyst 51 using the output signal of the air-fuel ratio sensor 6 and controls the fuel injection quantity through the fuel injection valve 3 so as to adjust the air-fuel ratio of the exhaust gas flowing into the three-way catalyst 51 to a target air-fuel ratio (e.g. the stoichiometric air-fuel ratio).

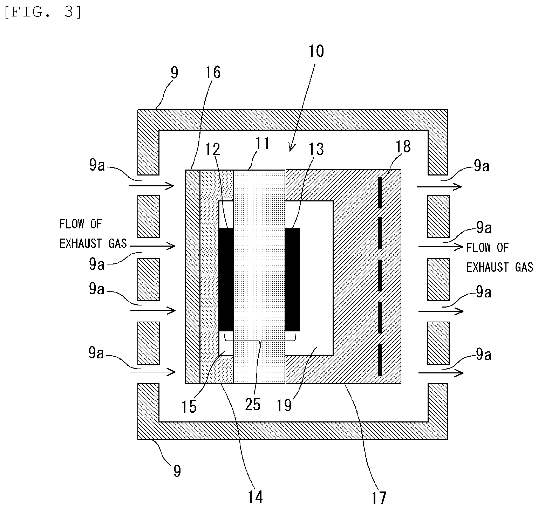

Now, the structure of the air-fuel ratio sensor 6 will be briefly described with reference to Figs. 2 and 3. Fig. 2 is an enlarge cross sectional view schematically showing the portion around the air-fuel ratio sensor 6 shown in Fig. 1. Fig. 3 is a cross sectional view taken along line A-A' in Fig. 2.

As shown in Fig. 2, the air-fuel ratio sensor 6 includes a main sensor unit 10, which will be specifically described later, and a protective cover 9. The protective cover 9 is a heat-resisting housing that enclose the main sensor unit 10, and a part of the protective cover is exposed to the exhaust passage 5. Enclosing the main sensor unit 10 by the protective cover 9 reinforces its mechanical strength.

As shown in Fig. 3, the protective cover 9 has a plurality of air holes 9a, which provide communication between its interior and exterior. Thus, the air-fuel ratio sensor 6 is structured in such a way that a part of the exhaust gas flowing in the exhaust passage 5 passes through the air holes 9a of the protective cover 9 to reach the main sensor unit 10. Although the protective cover 9 shown in Fig. 3 is single-walled, it may alternatively be double-walled with walls having air holes 9a.

Now, the general configuration of the main sensor unit 10 will be described. The main sensor unit 10 includes a sensor element 11 made of an oxygen ion conductive solid electrolyte. For instance, the sensor element 11 is made of zirconium oxide (zirconia). On one side of the sensor element 11 is provided an exhaust gas side electrode 12, and on the other side of the sensor element 11 is provided an atmosphere side electrode 13. The exhaust gas side electrode 12 and the atmosphere side electrode 13 are made of a metal material having high catalytic activity, such as platinum. Thus, the sensor element 11 is sandwiched between the pair of electrodes, namely the exhaust gas side electrode 12 and the atmosphere side electrode 13.

On the side of the exhaust gas side electrode 12 facing away from the sensor element 11 is provided a diffusion rate limiting layer 14. The diffusion rate limiting layer 14 is arranged in such a way as to cover the exhaust gas side electrode 12 and the surface of the sensor element 11 on which the exhaust gas side electrode 12 is provided. The diffusion rate limiting layer 14 is a layer made of a porous material such as a ceramic material and has the function of limiting the rate of diffusion of the exhaust gas. In other words, the diffusion rate limiting layer 14 has appropriate porosity and density that allow various components in the exhaust gas to diffuse at an appropriate diffusion rate. On the side of the diffusion rate limiting layer 14 facing away from the sensor element 11 is provided a protective layer 16. The sensor element 11 and the diffusion rate limiting layer 14 define a gas chamber 15 therebetween. The exhaust gas enters the gas chamber 15 through the diffusion rate limiting layer 14. Since the exhaust gas side electrode 12 is disposed in the gas chamber 15, the exhaust gas side electrode 12 is exposed to the exhaust gas through the diffusion rate limiting layer 14. The provision of the gas chamber 15 is not essential. The diffusion rate limiting layer 14 may be in direct contact with the surface of the exhaust gas side electrode 12.

On the other side of the sensor element 11 is provided a heater layer 17, in which a heater 18 is embedded. The heater 18 is capable of heating the main sensor unit 10 to a desired active temperature (e.g. 700°C) with supply of electrical power from an external electrical circuit (not shown). This electrical circuit is electrically connected with the ECU 20, and the electrical power supplied to the heater 18 is controlled by the ECU 20. The sensor element 11 and the heater layer 17 define an atmosphere chamber 19 therebetween. The atmosphere chamber 19 is in communication with the atmosphere through atmosphere holes (not shown), so that the atmosphere side electrode 13 is continuously exposed to the atmosphere, though the air-fuel ratio sensor 6 is disposed in the exhaust passage 5. In this embodiment, the above-described main sensor unit 10 corresponds to the measuring part according to the present invention.

Now, the principle of measurement of the air-fuel ratio of the exhaust gas by the air-fuel ratio sensor will be described. The exhaust gas entering inside the protective cover 9 through air holes 9a flows into the diffusion rate limiting layer 14 and travels inside the diffusion rate limiting layer 14 while diffusing toward the exhaust gas side electrode 12. The exhaust gas contains reducers such as CO and HC and oxidizers such as O₂ and NOx. These components react with each other while they travel to the surface of the exhaust gas side electrode 12 and/or after they reach the exhaust gas side electrode 12 until equilibrium is established. When the air-fuel ratio of the exhaust gas is the stoichiometric air-fuel ratio, the oxidizers and reducers will be both extinguished. In contrast, when the air-fuel ratio of the exhaust gas is richer than the stoichiometric air-fuel ratio, reducers will remain. Likewise, when the air-fuel ratio of the exhaust gas is leaner than the stoichiometric air fuel ratio, oxidizers will remain.

In the following, the portion including the exhaust gas side electrode 12, the atmosphere side electrode 13 and the sensor element 11 sandwiched between them will be referred to as the "cell 25". In this embodiment, a voltage is applied between the exhaust gas side electrode 12 and the atmosphere side electrode 13 via a power supply line not shown in the drawings. When oxidizers remain in the exhaust gas that reaches the surface of the exhaust gas side electrode 12, in other words when the air-fuel ratio of the exhaust gas is leaner than the stoichiometric air-fuel ratio, while a voltage is applied between the electrodes, oxygen in the exhaust gas is ionized, and oxygen ions propagate from the exhaust gas side electrode 12 to the atmosphere side electrode 13 through the sensor element 11, resulting in an electrical current flowing in the cell 25. When reducers remain around the exhaust gas side electrode 12, in other words when the air-fuel ratio of the exhaust gas is richer than the stoichiometric air-fuel ratio, oxygen in the atmosphere is ionized, and oxygen ions propagate from the atmosphere side electrode 13 to the exhaust gas side electrode 12 through the sensor element 11 and reacts with the reducers, resulting in an electrical current flowing in the cell 25 in the direction reverse to that in the above case. When the air-fuel ratio of the exhaust gas is the stoichiometric air-fuel ratio, oxygen ions basically do not propagate between the electrodes through the sensor element 11, and no electrical current flows in the cell 25.

As the diffusion rate of the exhaust gas is limited by the diffusion rate limiting layer 14, there is a range of applied voltage in which the current is saturated at a constant value even if the applied voltage is increased. This electrical current value is called the limiting current value. When the air-fuel ratio of the exhaust gas is leaner than the stoichiometric air-fuel ratio, the electrical current is saturated at the limiting current value because the diffusion rate of oxidizers toward the exhaust gas side electrode 12 is limited by the diffusion rate limiting layer. When the air-fuel ratio of the exhaust gas is richer than the stoichiometric air-fuel ratio, the electrical current is saturated at the limiting current value because the diffusion rate of reducers toward the exhaust gas side electrode 12 is limited by the diffusion rate limiting layer. The air-fuel ratio of the exhaust gas can be determined by measuring this limiting current value.

If particulate matter, oil components or the like adhere to the air holes 9a of the protective cover 9 of this air-fuel ratio sensor 6, the quantity of exhaust gas flowing into the interior of the protective cover 9 per unit time is apt to decrease. When the quantity of exhaust gas flowing into the interior of the protective cover 9 per unit time decreases, if the concentrations of components such as O₂, HC, CO, and NOx in the exhaust gas discharged from the internal combustion engine 1 change depending on the operation state of the internal combustion engine 1, it takes some time until the concentrations of these components in the exhaust gas inside the protective cover 9 change to the concentrations that correspond to the operation state of the internal combustion engine 1 (namely, the concentrations in the exhaust gas discharged from the internal combustion engine 1). Therefore, until the concentrations of components such as O₂, HC, CO, and NOx in the exhaust gas inside the protective cover 9 change to the concentrations corresponding to the operation state of the internal combustion engine 1, the output of the air-fuel ratio sensor 6 does not agree with the concentrations corresponding to the operation state of the internal combustion engine 1. This means that the responsiveness of the air-fuel ratio sensor 6 is deteriorated.

If particulate matter, oil components or the like adhere to the diffusion rate limiting layer 14 in the air-fuel ratio sensor 6, the porosity of the diffusion rate limiting layer 14 is apt to decrease. On the other hand, if the diffusion rate limiting layer 14 is deteriorated, the porosity of the diffusion rate limiting layer 14 is apt to increase. When the porosity of the diffusion rate limiting layer 14 becomes lower, the resistance against the flow of exhaust gas through the diffusion rate limiting layer 14 becomes higher than when the porosity is high. Hence, the lower the porosity of the diffusion rate limiting layer 14 is, the smaller the current value at which the electrical current flowing in the cell 25 is saturated tends to be. In other words, the lower the porosity of the diffusion rate limiting layer 14 is, the more the limiting current value is apt to decrease.

The output of the above-described air-fuel ratio sensor 6 is combinedly affected by the change in the responsiveness of the air-fuel ratio sensor 6 (i.e. the change in the time taken for the output of the air-fuel ratio sensor 6 to converge) and the change in the limiting current value. In the following, we will describe how the output of the air-fuel ratio sensor 6 changes with time when the oxygen concentration in the exhaust gas changes. In this embodiment, the ECU 20 changes the oxygen concentration in the exhaust gas by performing a fuel cut process. In this embodiment, the fuel cut process corresponds to the oxygen concentration control according to the present invention, and the ECU 20 functions as the control unit according to the present invention in performing the fuel cut process.

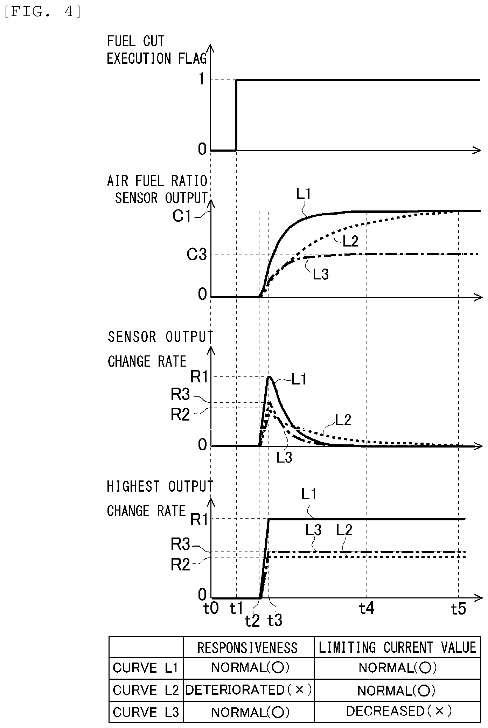

Fig. 4 shows changes with time of a fuel cut execution flag, the output of the air-fuel ratio sensor 6, the change rate of the output of the air-fuel ratio sensor 6 (which will also be referred to as the "sensor output change rate" hereinafter), and the highest value of the change rate of the output of the air-fuel ratio sensor 6 (which will also be referred to as the "highest output change rate" hereinafter) when the fuel cut process is performed in the internal combustion engine 1. The sensor output change rate is the change in the output of the air-fuel ratio sensor 6 per unit time. In the process shown in Fig. 4, the ECU 20 controls the fuel injection quantity through the fuel injection valve 3 so that the air-fuel ratio of the exhaust gas flowing into the three-way catalyst 51 is made equal to the stoichiometric air-fuel ratio, during the normal operation. In the graphs showing the changes with time of the output of the air-fuel ratio sensor 6, the sensor output change rate, and the highest output change rate in Fig. 4, the solid curves L1 represent the changes with time of the respective values in an air-fuel ratio sensor 6 with normal responsiveness and normal limiting current value (which will also be referred to as the "normal sensor" hereinafter), the broken curves L2 represent the changes with time of the respective values in an air-fuel ratio sensor 6 with normal limiting current value and abnormal responsiveness (which will also be referred to as the "abnormal response sensor" hereinafter), and the dot-and-dash curves L3 represent the changes with time of the respective values in an air-fuel ratio sensor 6 with normal responsiveness and decreased limiting current value (which will also be referred to as the "decreased output sensor" hereinafter).

In the control shown in Fig. 4, the internal combustion engine 1 is in normal operation from time t0 to time t1. During this period, the air-fuel ratio of the exhaust gas is the stoichiometric air fuel ratio, and the output of the air-fuel ratio sensor 6 is 0. The fuel cut execution flag changes from 0 to 1 at time t1, and the ECU 20 performs the fuel cut process. Then, after the lapse of a certain delay time, the air-fuel ratio of the exhaust gas becomes leaner than the stoichiometric air fuel ratio. As this exhaust gas passes through the air holes 9a of the protective cover 9 of the air-fuel ratio sensor 6 to reach the main sensor unit 10, the output of the air-fuel ratio sensor 6 starts to increase from 0.

In the course of the increase of the output of the air-fuel ratio sensor 6, the increase of the output tends to be relatively slow in a period immediately after the start of the increase of the output, and after the lapse of some time since the start of increase of the output, the output tends to increase greatly. Thus, as shown by lines L1, L2, and L3 in Fig. 4, the output of the air-fuel ratio sensor 6 starts to increase from 0 at time t2, and the sensor output change rate increases thereafter to reach R1 in the case of the normal sensor, R2 in the case of abnormal response sensor, and R3 in the case of the decrease output sensor, at time t3. After time t3, the sensor output change rate decreases with time in all the sensors, and the sensor output converges to C1 at time t4 in the case of the normal sensor, to C3 at time t4 in the case of the decreased output sensor, and to C1 at time t5 in the case of the abnormal response sensor.

In the course of change of the output of the air-fuel ratio sensor 6, the period from the time when the output of the air-fuel ratio sensor 6 starts to change with the performance of the fuel cut process to the time when the sensor output converges will be hereinafter referred to as the "reference period". In the case of the abnormal response sensor, the reference period is longer than that of the normal sensor and the decreased output sensor. This indicates that the responsiveness of the abnormal response sensor is deteriorated. As to the converged sensor output value with the reference period, the converged value C3 of the decreased output sensor is smaller than the converged value C1 of the normal sensor and the abnormal response sensor. This indicates that the limiting current value of the decreased output sensor is decreased.

As shown in Fig. 4, the sensor output change rate at time t3 is the highest output change rate during the reference period. The value R2 of the highest output change rate of the abnormal response sensor is lower than the value R1 of the normal sensor. This is because the time taken for the sensor output to change from 0 to C1 is longer in the abnormal response sensor than in the normal sensor. In other words, the lower the responsiveness is, the lower the highest output change rate is. Therefore, it may be considered that the responsiveness of the air-fuel ratio sensor 6 can be estimated on the basis of the highest output change rate.

However, the value R3 of the highest output change rate of the decreased output sensor is also lower than the value R1 of the normal sensor. This is because the converged sensor output value (C3) of the decreased output sensor is smaller than the converged sensor output value (C1) of the normal sensor, though the time taken for the sensor output to converge is equal between the sensors. Thus, the highest output change rate of the decreased output sensor is lower than that of the normal sensor, though the responsiveness of the decreased output sensor is not deteriorated. Therefore, estimating the responsiveness of the air-fuel ratio sensor 6 only on the basis of the sensor output change rate can result in degraded estimation accuracy.

To avoid the above problem, the ECU 20 is configured to calculate a responsiveness index that correlates with the responsiveness of the air-fuel ratio sensor 6. Specifically, the ECU 20 calculates the responsiveness index by dividing the largest value of the absolute value of the output change rate of the air-fuel ratio sensor 6 (which will also be referred to as the "absolute change rate" hereinafter) during the reference period from the time when the fuel cut process is started to the time when the output of the air-fuel ratio sensor 6 changing with the performance of the fuel cut process converges by the difference between the output value of the air-fuel ratio sensor 6 at the time when the absolute change rate becomes largest and the converged value of the output of the air-fuel ratio sensor 6 with the aforementioned reference period. The ECU 20 estimates the responsiveness of the air-fuel ratio sensor 6 on the basis of this responsiveness index. The ECU 20 functions as the calculation unit according to the present invention in calculating the responsiveness index and as the responsiveness diagnosis unit according to the first aspect of the present invention in estimating the responsiveness of the air-fuel ratio sensor 6.

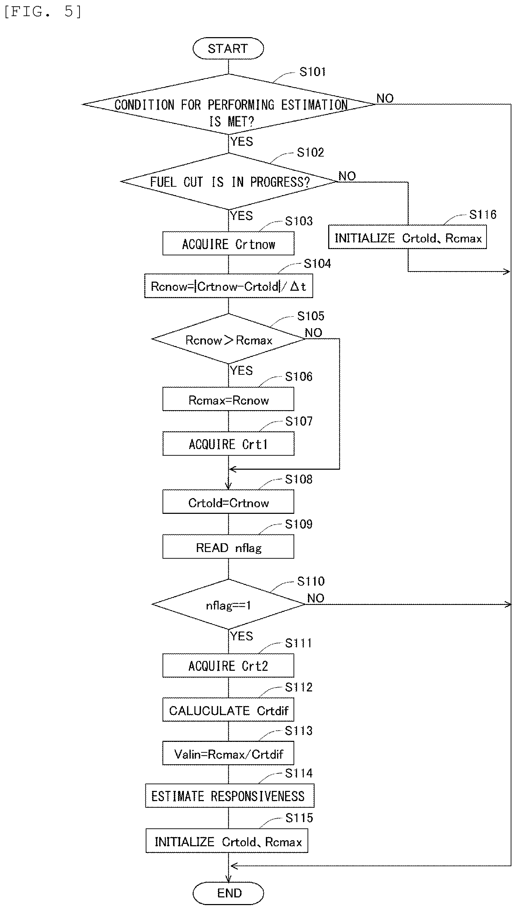

In the following, a control flow executed by the ECU 20, which serves as the diagnosis apparatus for an exhaust gas sensor according to the present invention, will be described with reference to Fig. 5. Fig. 5 is a flow chart of the control process according to the embodiment of the first aspect of the present invention. In this embodiment, this flow is executed by the ECU 20 repeatedly at regular intervals during the operation of the internal combustion engine 1. In this embodiment, this flow is executed at a predetermined interval Δt.

In this processing flow, firstly in step S101, it is determined whether or not a condition for performing the estimation of the responsiveness of the air-fuel ratio sensor 6 is met. In step S101, an affirmative determination is made when, for example, the vehicle provided with the internal combustion engine 1 has travelled a predetermined distance, the internal combustion engine 1 has operated for a predetermined length of time, or the internal combustion engine 1 has been stopped and restarted afterward, after the responsiveness of the air-fuel ratio sensor 6 was estimated last time. The above conditions are merely for illustrative purposes, and a determination in step S101 as to whether or not the condition for performing the estimation of the responsiveness of the air-fuel ratio sensor 6 is met may be made based on other known technologies. If an affirmative determination is made in step S101, the ECU 20 executes the processing of step S102 next. If a negative determination is made in step S101, the execution of this flow is terminated.

If an affirmative determination is made in step S101, then in step S102 it is determined whether or not the fuel cut process is in progress. As described above, in the case, for example, where the ECU 20 is controlling the fuel injection quantity through the fuel injection valve 3 so that the air-fuel ratio of the exhaust gas flowing into the three-way catalyst 51 is made equal to the stoichiometric air-fuel ratio during the normal operation, the air-fuel ratio of the exhaust gas changes from the stoichiometric air-fuel ratio to air-fuel ratios leaner than the stoichiometric air-fuel ratio during the fuel cut process. As the oxygen concentration in the exhaust gas changes in this way, the output of the air-fuel ratio sensor 6 changes. This enables calculation of the responsiveness index Valin that will be described later. If an affirmative determination is made in step S102, the ECU 20 executes the processing of step S103 next. If a negative determination is made in step S102, the ECU 20 executes the processing of step S116 next.

If an affirmative determination is made in step S102, then in step S103 the present value Crtnow of the output of the air-fuel ratio sensor 6 (which will also be referred to as the "present output value" hereinafter) is acquired. In step S103, the present limiting current value corresponding to the air-fuel ratio of the exhaust gas that reaches the main sensor unit 10 of the air-fuel ratio sensor 6 is acquired as the present output value Crtnow.

Then, in step S104, the present value Rcnow of the absolute change rate is calculated. In step S104, the present value Rcnow of the absolute change rate is calculated by dividing the absolute value of the difference between the present output value Crtnow acquired in step S103 and the previous value Crtold of output of the air-fuel ratio sensor 6 (which will also be referred to as the "previous output value" hereinafter) by the predetermined interval Δt. The previous output value Crtold is a value that is updated by the processing of step S108, which will be described later, or initialized by the processing of step S115, which will be described later, and stored in the ROM of the ECU 20.

Then, in step S105, it is determined whether or not the present value Rcnow of the absolute change rate calculated in step S104 is larger than the largest value Rcmax of the absolute change rate. The largest value Rcmax of the absolute change rate is updated by the processing of step S106, which will be described later or initialized by the processing of step S115, which will be described later, and stored in the ROM of the ECU 20. If an affirmative determination is made in step S105, the ECU 20 executes the processing of step S106 next. If a negative determination is made in step S105, the ECU 20 executes the processing of step S108.

If an affirmative determination is made in step S105, then in step S106 the largest value Rcmax of the absolute change rate is updated. Specifically, in step S106, the largest value Rcmax of the absolute change rate is updated to the present value Rcnow of the absolute change rate calculated in step S104. Then in step S107, the output value Crt1 of the air-fuel ratio sensor 6 at the time when the absolute change rate becames largest is acquired. This output value Crt1 will also be referred to as the "first output value" hereinafter. In step S107, the present output value Crtnow at that time is acquired as the first output value Crt1.

Then, in step S108, the previous output value Crtold is updated. Specifically, in step S108, the previous output value Crtold is updated to the present output value Crtnow acquired in step S103.

Then in step S109, a convergence determination flag nflag about the determination as to the convergence of the output value of the air-fuel ratio sensor 6 is read. The convergence determination flag nflag is a flag that is set to 1 when it is determined that the output value of the air-fuel ratio sensor 6 has converged. The value of the convergence determination flag nflag is set by a known flow other than this flow and stored in the ROM of the ECU 20. In step S109, the convergence determination flag nflag stored in the ROM of the ECU 20 is read. The convergence determination flag nflag is set to 1 when, for example, the present value Rcnow of the absolute change rate that had once increased after the start of the fuel cut process becomes equal to or smaller than a predetermined value close to zero.

Then in step S110, it is determined whether or not the convergence determination flag nflag read in step S109 is 1. An affirmative determination is made in step S110 when it is determined that the output value of the air-fuel ratio sensor 6 has converged. In that case, the ECU 20 executes the processing of step S111 next. On the other hand, a negative determination is made in step S110 when it is determined that the output value of the air-fuel ratio sensor 6 has not converged yet. In that case, the execution of this flow is terminated. Since this processing flow is executed repeatedly at a predetermined interval Δt as described above, even when the execution of this flow is terminated pursuant to a negative determination made in step S110 this time, the responsiveness of the air-fuel ratio sensor 6 will be estimated in the processing of step S114 (described later) in the next or subsequent execution of this flow, if an affirmative determination is made in step S110 in the next or subsequent execution of this flow.

If an affirmative determination is made in step S110, then in step S111, the converged value Crt2 of the output of the air-fuel ratio sensor 6 is acquired. This converged value Crt2 will also be referred to as the "second output value" hereinafter. In step S111, the second output value Crt2 may be obtained using a known method. Then, in step S112, the difference Crtdif between the first output value Crt1 and the second output value Crt2 is calculated.

Then, in step S113, the responsiveness index Valin is calculated. In step S113, specifically, the responsiveness index Valin is calculated by dividing the largest value Rcmax of the absolute change rate by the difference Crtdif calculated in step S112. Then, in step S114, the responsiveness of the air-fuel ratio sensor 6 is estimated on the basis of the responsiveness index Valin. In step S114, the responsiveness of the air-fuel ratio sensor 6 may be estimated higher when the responsiveness index Valin is large than when it is small.

Then, in step S115, the previous output value Crtold and the largest value Rcmax of the absolute change rate are initialized. In this flow, the previous output value Crtold is initialized to the limiting current value corresponding to the air-fuel ratio of the exhaust gas during the normal operation of the internal combustion engine 1. For example, when the ECU 20 is controlling the fuel injection quantity through the fuel injection valve 3 so that the air-fuel ratio of the exhaust gas flowing into the three-way catalyst 51 is made equal to the stoichiometric air-fuel ratio during the normal operation, the previous output value Crtold is initialized to 0. The largest value Rcmax of the absolute change rate is also initialized to 0. After the completion of the processing of step S115, the execution of this flow is ended.

If a negative determination is made in step S102, then in step S116, the previous output value Crtold and the largest value Rcmax of the absolute change rate are initialized. Thus, even if the fuel cut process ends before the estimation of the responsiveness of the air-fuel ratio sensor 6 is completed, the previous output value Crtold and the largest value Rcmax of the absolute change rate are initialized. The processing of step S116 is substantially the same as the above-described processing of step S115. After the completion of the processing of step S116, the execution of this flow is ended.

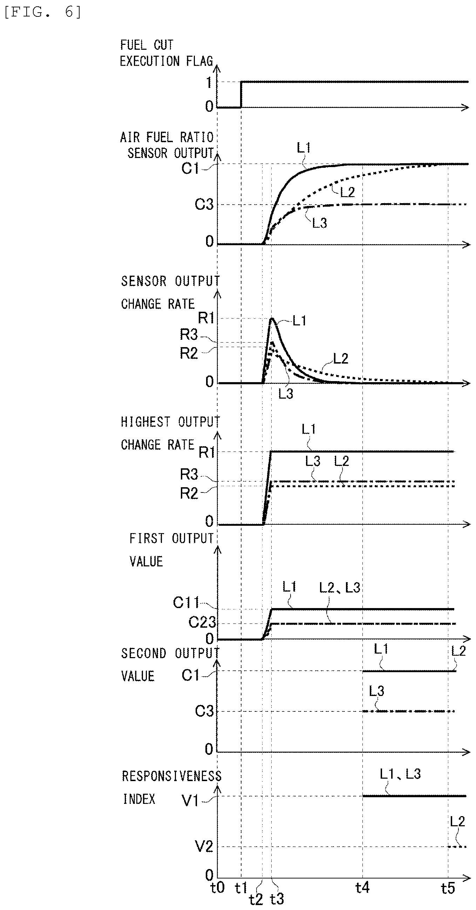

In the following, the responsiveness index Valin calculated as above will be described with reference to the time chart. Fig. 6 shows changes with time of the fuel cut execution flag, the output of the air-fuel ratio sensor 6, the sensor output change rate, and the highest output change rate, as with Fig. 4. Moreover, Fig. 6 shows the changes with time of the first output value Crt1, the second output value Crt2, and the responsiveness index Valin.

As shown in Fig. 6, at time t3, the first output value Crt1 of the normal sensor is C11, and the first output value Crt1 of the abnormal response sensor and the decreased output sensor is C23. Although the first output value Crt1 of the abnormal response sensor and the first output value Crt1 of the decreased output sensor can be different values, these values are substantially equal in the case shown in Fig. 6. Therefore, the first output value Crt1 of the abnormal response sensor and the first output value Crt1 of the decreased output sensor are both indicated as C23 in Fig. 6. At time t4 at which the output of the normal sensor and the decreased output sensor converges, the second output value Crt2 of the normal sensor is C1, and the second output value Crt2 of the decreased output sensor is C3. At time t5 at which the output of the abnormal response sensor converges, the second output value Crt2 of the abnormal response sensor is C1.

In the case of the normal sensor and the decreased output sensor, the responsiveness index Valin is calculated when the sensor output converges at time t4. In the case of the abnormal response sensor, the responsiveness index Valin is calculated when the sensor output converges at time t5. As shown in Fig. 6, while the value of the responsiveness index Valin of the normal sensor and the decreased output sensor is V1, the value of the responsiveness index Valin of the abnormal response sensor is V2, which is smaller than V1. Thus, the value of the responsiveness index Valin of only the abnormal response sensor with deteriorated responsiveness is lower than the value of the responsiveness index Valin of the normal sensor.

The diagnosis apparatus for an exhaust gas sensor according to the first aspect of the present invention is capable of estimating the responsiveness of the air-fuel ratio sensor 6 with as high accuracy as possible by calculating the responsiveness index Valin in the above described way and estimating the responsiveness of the air-fuel ratio sensor 6 on the basis of the responsiveness index Valin thus calculated.

### < First Modification >

A first modification of the above-described first embodiment will be described. The components and processing in this modification that are substantially the same as those in the above-described first embodiment will not be further described specifically.

In the above-described first embodiment, the ECU 20 changes the oxygen concentration in the exhaust gas by performing the fuel cut process. This is based on the fact that performing the fuel cut process causes the air-fuel ratio of the exhaust gas to change to air-fuel ratios leaner than before. When the fuel cut process is ended to return to the normal operation, the air-fuel ratio of the exhaust gas, which has been leaner than the stoichiometric air-fuel ratio during the fuel cut process, changes to richer air-fuel ratios. In this modification, the ECU 20 changes the oxygen concentration in the exhaust gas by performing the process of returning from the fuel cut process to the normal operation. In this embodiment, the returning process corresponds to the oxygen concentration control according to the present invention, and the ECU 20 functions as the control unit according to the present invention in performing the returning process.

A control flow in this modification will be described with reference to the flow chart of Fig. 5. In the control flow in this modification, if an affirmative determination is made in step S101, the processing of determining whether or not the process of returning from the fuel cut process to the normal operation is in progress is executed in place of the processing of step S102. The process of returning from the fuel cut process to the normal operation continues from the time when the ECU 20 starts this returning process until a certain period of time elapses. This period of time is determined in advance. This period of time is determined in such a way that the output values of the air-fuel ratio sensor 6, which changes with the returning process, converges within this period of time. In this modification, the output value of the air-fuel ratio sensor 6 converges, for example, to an output value corresponding to the air-fuel ratio during the normal operation. During the process of returning from the fuel cut process to the normal operation, the air-fuel ratio of the exhaust gas, which has been leaner than the stoichiometric air-fuel ratio during the fuel cut process, changes to richer air-fuel ratios. Consequently, the oxygen concentration in the exhaust gas changes, so that the output of the air-fuel ratio sensor 6 changes, enabling calculation of the responsiveness index Valin. If an affirmative determination is made in the above determination process, the ECU 20 executes the processing of step S103 next. If a negative determination is made in the above determination process, the ECU 20 executes the processing of step S116.

As described above, the responsiveness index Vlin can be calculated during the process of returning from the fuel cut process to the normal operation. The responsiveness of the air-fuel ratio sensor 6 can be estimated with as high accuracy as possible by estimating the responsiveness of the air-fuel ratio sensor 6 on the basis of the responsiveness index Valin.

### < Second Modification >

In the following, a second modification of the above-described first embodiment will be described. The components and processing in the second modification that are substantially the same as those in the above-described first embodiment will not be further described specifically.

In the above-described first embodiment, the ECU 20 changes the oxygen concentration in the exhaust gas by performing the fuel cut process. In this modification, the ECU 20 changes the oxygen concentration in the exhaust gas by performing an air-fuel ratio control for changing the air-fuel ratio for the purpose of estimating the responsiveness of the air-fuel ratio sensor 6. In this air-fuel ratio control, for example, the air-fuel ratio that is richer than the stoichiometric air-fuel ratio is changed to an air-fuel ratio leaner than the stoichiometric air-fuel ratio. In this modification, the air-fuel ratio control corresponds to the oxygen concentration control according to the present invention, and the ECU 20 functions as the control unit according to the present invention in performing the air-fuel ratio control.

A control flow in this modification will be described with reference to the flow chart in Fig. 5. In the control flow in this modification, if an affirmative determination is made in step S101, the processing of determining whether or not the air-fuel control is being performed is executed in place of the processing of step S102. While the air-fuel ratio control is being performed, the oxygen concentration in the exhaust gas changes, so that the output of the air-fuel ratio sensor 6 changes, enabling calculation of the responsiveness index Valin. In this modification, since the air-fuel ratio richer than the stoichiometric air-fuel ratio is changed to an air-fuel ratio leaner than the stoichiometric air-fuel ratio, the output value of the air-fuel ratio 6 converges to an output value corresponding to this lean air fuel ratio. If an affirmative determination is made in the above determination process, the ECU 20 executes the processing of step S103 next. If a negative determination is made in the above determination process, the ECU 20 executes the processing of step S116.

As described above, the responsiveness index Vlin can be calculated while the air-fuel ratio control performed. The responsiveness of the air-fuel ratio sensor 6 can be estimated with as high accuracy as possible by estimating the responsiveness of the air-fuel ratio sensor 6 on the basis of the responsiveness index Valin.

### < Second Embodiment >

In the following, a second embodiment of the present invention will be described. The components and processing in the second embodiment that are substantially the same as those in the above-described first embodiment will not be further described specifically.

In the above-described first embodiment, the responsiveness of the air-fuel ratio sensor 6 is estimated on the basis of the responsiveness index. In the second embodiment, the ECU 20 uses the responsiveness index to diagnose abnormality in the responsiveness of the air-fuel ratio sensor 6. In diagnosing abnormality in the responsiveness of the air-fuel ratio sensor 6, the ECU 20 functions as the abnormality diagnosis unit according to the second aspect of the present invention.

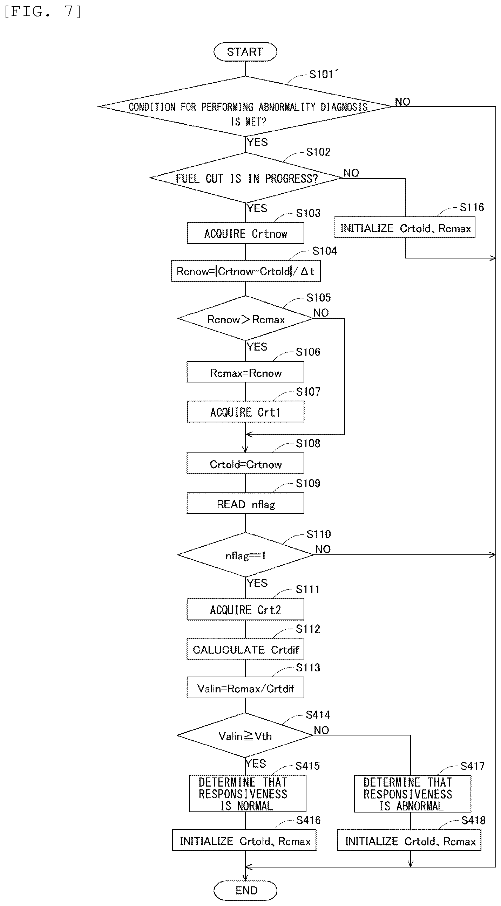

In the following, a control flow in the second embodiment will be described with reference to Fig. 7. Fig. 7 is a flow chart of the control flow according to the embodiment of the second aspect of the present invention. In the process shown in Fig. 7, the processing of steps S414 through S418 is executed in place of the processing of step S114 and S115 in the flow chart of Fig. 5.

In step S101' in the control flow shown in Fig. 7, it is determined whether or not a condition for performing diagnosis of abnormality in the responsiveness of the air-fuel ratio sensor 6 is met. In step S101', an affirmative determination is made when, for example, the vehicle provided with the internal combustion engine 1 has travelled a predetermined distance, the internal combustion engine 1 has operated for a predetermined length of time, or the internal combustion engine 1 has been stopped and restarted afterward, after the abnormality diagnosis was performed last time. The above conditions are merely for illustrative purposes, and a determination as to whether or not the condition for performing diagnosis of abnormality in the responsiveness of the air-fuel ratio sensor 6 is met may be made based on other known technologies. If an affirmative determination is made in step S101', the ECU 20 executes the processing of step S102 next. If a negative determination is made in step S101', the execution of this flow is terminated.

In the control flow shown in Fig. 7, after the processing of step S113, it is determined in step S414 whether or not the responsiveness index Valin calculated in step S113 is equal to or larger than a determination threshold Vth. As described above, the responsiveness index Valin correlates with the responsiveness of the air-fuel ratio sensor 6. Therefore, abnormality in the responsiveness of the air-fuel ratio sensor 6 can be diagnosed by comparing the responsiveness index Valin with the determination threshold Vth. The determination threshold Vth is a threshold for determining whether the air-fuel ratio sensor 6 has an abnormality in its responsiveness. Specifically, when the responsiveness index Valin is smaller than the determination threshold Vth, it may be determined that the air-fuel ratio sensor 6 has an abnormality in responsiveness. The determination threshold Vth is stored in the ROM of the ECU 20 in advance. If an affirmative determination is made in step S414, the ECU 20 executes the processing of step S415 next. If a negative determination is made in step S414, the ECU 20 executes the processing of step S417.

If an affirmative determination is made in step S414, then in step S415, it is determined that the responsiveness of the air-fuel ratio sensor 6 is normal. Then, in step S416, the previous output value Crtold and the largest value Rcmax of the absolute change rate are initialized. The processing of step S416 is substantially the same as the above-described processing of step S115. After the completion of the processing of step S416, the execution of this flow is ended.

If a negative determination is made in step S414, then in step S417, it is determined that the air-fuel ratio sensor 6 has an abnormality in its responsiveness. Then, in step S418, the previous output value Crtold and the largest value Rcmax of the absolute change rate are initialized. The processing of step S418 is substantially the same as the above-described processing of step S115. After the completion of the processing of step S418, the execution of this flow is ended.

As described above, by diagnosing abnormality of the air-fuel ratio sensor 6 on the basis of the responsiveness index Valin, abnormality in the responsiveness of the air-fuel ratio sensor 6 can be determined with improved accuracy.

### < Third Embodiment >

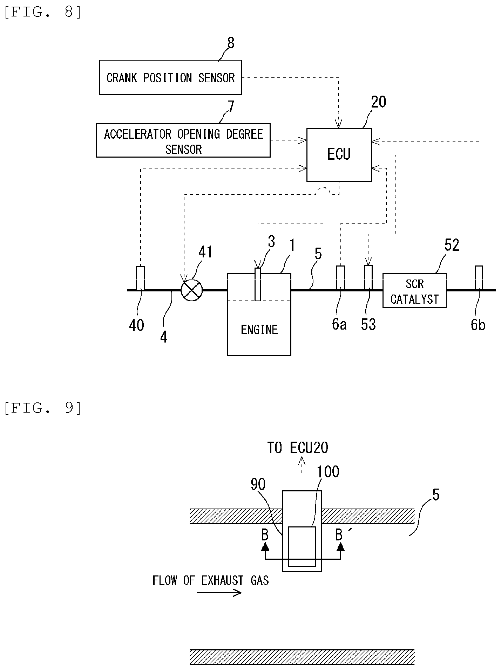

In the following a third embodiment of the present invention will be described. Fig. 8 is a diagram showing the general configuration of the air-intake and exhaust systems of an internal combustion engine according to the third embodiment. The internal combustion engine 1 shown in Fig. 8 is a compression-ignition internal combustion engine (diesel engine), which has a fuel injection valve 3 that injects fuel into the cylinder. The components and processing in the third embodiment that are substantially the same as those in the above-described first embodiment will not be further described specifically.

The exhaust passage 5 of the internal combustion engine 1 is provided with a selective catalytic reduction NOx catalyst 52, which will be also referred to as the "SCR catalyst 52" hereinafter. The exhaust passage 5 is also provided with a urea solution addition valve 53 arranged upstream of the SCR catalyst 52. The urea solution addition valve 53 supplies urea as a precursor of ammonia to the SCR catalyst 52. The urea thus supplied is hydrolyzed to produce ammonia, which is adsorbed by the SCR catalyst 52. The ammonia adsorbed by the SCR catalyst 52 serves as a reducing agent to reduce NOx in the exhaust gas. The exhaust passage 5 is provided with a first NOx sensor 6a arranged upstream of the SCR catalyst 52. The exhaust passage 5 is provided with a second NOx sensor 6b arranged downstream of the SCR catalyst 52. Details of these NOx sensors will be described later. In this embodiment, the first NOx sensor 6a and the second NOx sensor 6b correspond to the exhaust gas sensor according to the present invention.

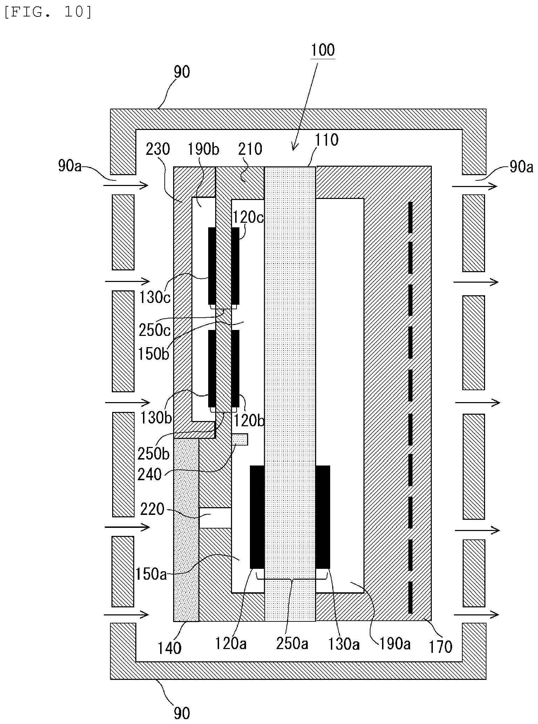

Now, the structure of the first NOx sensor 6a and the second NOx sensor 6b will be described briefly with reference to Figs. 9 and 10. Fig. 9 is an enlarge cross sectional view schematically showing the portion around the first NOx sensor 6a shown in Fig. 8. Fig. 10 is a cross sectional view taken along line B-B' in Fig. 9. The first NOx sensor 6a and the second NOx sensor 6b have the same structure, and the first NOx sensor 6a and the second NOx sensor 6b will be collectively referred to as the "NOx sensor" hereinafter.

As with the air-fuel ratio sensor 6 in the first embodiment, the NOx sensor used in this embodiment includes a main sensor unit 100 and a protective cover 90. The protective cover 90 has a plurality of air holes 90a. Although the protective cover 90 shown in Fig. 10 is single-walled, it may alternatively be double-walled with walls having air holes 90a.

The general configuration of the main sensor unit 100 will be described. As with the main sensor unit 10 of the air-fuel ratio sensor 6 in the first embodiment, the main sensor unit 100 of the NOx sensor used in this embodiment includes exhaust gas side electrodes 120a, 120b, 120c exposed to the exhaust gas, atmosphere side electrodes 130a, 130b, 130c exposed to the atmosphere, sensor elements 110, 210 sandwiched between the exhaust gas side electrodes and the atmosphere side electrodes, and a diffusion rate limiting layer 140 made of a porous material that limits the rate of diffusion of the exhaust gas delivered to the exhaust gas side electrodes. As shown in Fig. 10, there are a plurality of regions each of which includes an exhaust gas side electrode, an atmosphere side electrode, and a sensor element sandwiched between them. These regions will be referred to as "pump cell 250a", "monitor cell 250b", and "sensor cell 250c".

One sensor element 110 and the other sensor element 210 define a first gas chamber 150a and a second gas chamber 150b therebetween. The exhaust gas enters the first gas chamber 150a through the diffusion rate limiting layer 140 and a pin hole 220. The sensor element 110 and a heater layer 170 define a first atmosphere chamber 190a therebetween, which is in communication with the atmosphere. When a voltage is applied between the exhaust gas side electrode 120a and the atmosphere side electrode 130a in the pump cell 250a, oxygen in the first gas chamber 150a is discharged to the first atmosphere chamber 190a by the pumping effect.

The exhaust gas passing the region near the pump cell 250a flows into the second gas chamber 150b, which is in communication with the first gas chamber 150a via a diaphragm 240. When oxygen in the first gas chamber 150a is discharged to the first atmosphere chamber 190a by the pump cell 250a, the second gas chamber 150b is brought into a specific low oxygen concentration state. On the sensor element 210 is provided a spacer 230 to form a second atmosphere chamber 190b between the sensor element 210 and the spacer 230. The second atmosphere chamber 190b is in communication with the atmosphere. When a voltage is applied to the exhaust gas side electrode 120b and the atmosphere side electrode 130b of the monitor cell 250b, oxygen remaining in the second gas chamber 150b is transferred to the second atmosphere chamber 190b by the pumping effect. The exhaust gas side electrode 120b of the monitor cell 250b is inactive to reductive decomposition of NOx. Therefore, an output current that correlates with the concentration of remaining oxygen is produced.

Among the exhaust gas side electrode 120c and the atmosphere side electrode 130c of the sensor cell 250c, the exhaust gas side electrode 120c is active to the reductive decomposition of NOx. When a voltage is applied to the exhaust gas side electrode 120c and the atmosphere side electrode 130c of the sensor cell 250c, NOx and oxygen remaining in the second gas chamber 150b is transferred to the second atmosphere chamber 190b by the pumping effect. Thus, an output current that correlates with NOx concentration and the concentration of remaining oxygen is produced. The NOx concentration can be determined by subtracting the output current of the monitor cell 250b from the output current of the sensor cell 250c.

As described above, in the NOx sensor according to this embodiment, the pump cell 250a outputs an electrical current correlating with the oxygen concentration in the first gas chamber 150a, the monitor cell 250b outputs an electrical current correlating with the oxygen concentration in the second gas chamber 150b, and the sensor cell 250c outputs an electrical current correlating with the NOx concentration and the oxygen concentration in the second gas chamber 150b. Thus, the NOx sensor used in this embodiment outputs an electrical current correlating with the oxygen concentration in the exhaust gas. Since the rate of diffusion of exhaust gas to the exhaust gas side electrodes 120a, 120b, 120c is limited by the diffusion rate limiting layer 140, there is a range of applied voltage in which the electrical current is saturated at a constant value even if the applied voltage is increased. Thus, there is a limiting current.

As in the case of the air-fuel ratio sensor 6 used in the first embodiment, if particulate matter, oil components or the like adhere to the air holes 90a of the protective cover 90 of this NOx sensor, there is a possibility that the responsiveness of the NOx sensor is deteriorated. Moreover, if particulate matter, oil components or the like adhere to the diffusion rate limiting layer 140, there is a possibility that the limiting current value may decrease. The output of the NOx sensor is combinedly affected by the change in the responsiveness of the NOx sensor (i.e. the change in the time taken for the output of the NOx sensor to converge) and the change in the limiting current value. Therefore, if the responsiveness of the NOx sensor is estimated only on the basis of the change rate of the output of the NOx sensor, the accuracy of the estimation may be deteriorated. Moreover, if a diagnosis as to the responsiveness of the NOx sensor is made only on the basis of the change rate of the output of the NOx sensor, there is a possibility that abnormality in the responsiveness of the sensor cannot be determined correctly.

In view of the above, the ECU 20 is configured to calculate a responsiveness index correlating with the responsiveness of the NOx sensor in a similar manner as in the case of the first embodiment. The ECU 20 calculates the responsiveness of the NOx sensor on the basis of this responsiveness index. Thus, the responsiveness of the NOx sensor can be estimated with as high accuracy as possible.

Moreover, the ECU 20 is configured to diagnose abnormality in the responsiveness of the NOx sensor on the basis of the responsiveness index, in a similar manner as in the case of the second embodiment. Thus, abnormality in the responsiveness of the NOx sensor can be determined with high accuracy as possible.

### Reference Signs List

- 1:: internal combustion engine
- 3:: fuel injection valve
- 4:: intake passage
- 5:: exhaust passage
- 6:: air-fuel ratio sensor
- 9:: protective cover
- 9a:: air hole
- 10:: main sensor unit
- 11:: sensor element
- 12:: exhaust gas side electrode
- 13:: atmosphere side electrode
- 14:: diffusion rate limiting layer
- 20:: ECU
- 40:: air flow meter
- 51:: three-way catalyst

## Claims

1. A diagnosis apparatus for an exhaust gas sensor applied to an internal combustion engine (1) comprising the exhaust gas sensor (6) to output an output signal representing the oxygen concentration in exhaust gas, the exhaust gas sensor (6) having a measuring part (10) that is configured to measure the oxygen concentration in the exhaust gas comprising a pair of electrodes including an exhaust gas side electrode (12) exposed to the exhaust gas and an atmosphere side electrode (13) exposed to the atmosphere and a porous diffusion rate limiting layer (14) that allows the exhaust gas to travel to said exhaust gas side electrode (12) while limiting the rate of diffusion of the exhaust gas delivered to said exhaust gas side electrode (12), and a protective cover (9) that is configured to enclose said measuring part (10) and including a plurality of air holes (9a), comprising:
a control unit (20) that is configured to perform oxygen concentration control to change the oxygen concentration in the exhaust gas;
a calculation unit (20) that is configured to calculate a responsiveness index correlating with the responsiveness of said exhaust gas sensor (6) by dividing the largest value of an absolute change rate defined as the absolute value of the change rate of said output signal during a reference period defined as a period from the time at which said oxygen concentration control starts to be performed to the time at which said output signal changing with the performance of said oxygen concentration control converges by the difference between the value of said output signal at the time when said absolute change rate becomes largest and a converged value of said output signal with said reference period; and
a responsiveness diagnosis unit (20) that is configured to estimate the responsiveness of said exhaust gas sensor (6) on the basis of said responsiveness index.

2. A diagnosis apparatus for an exhaust gas sensor applied to an internal combustion engine (1) comprising the exhaust gas sensor (6) to output an output signal representing the oxygen concentration in exhaust gas, the exhaust gas sensor (6) having a measuring part (10) that is configured to measure the oxygen concentration in the exhaust gas comprising a pair of electrodes including an exhaust gas side electrode (12) exposed to the exhaust gas and an atmosphere side electrode (13) exposed to the atmosphere and a porous diffusion rate limiting layer (14) that allows the exhaust gas to travel to said exhaust gas side electrode (12) while limiting the rate of diffusion of the exhaust gas delivered to said exhaust gas side electrode (12), and a protective cover (9) that is configured to enclose said measuring part (10) and including a plurality of air holes (9a), comprising:
a control unit (20) that is configured to perform oxygen concentration control to change the oxygen concentration in the exhaust gas;
a calculation unit (20) that is configured to calculate a responsiveness index correlating with the responsiveness of said exhaust gas sensor (6) by dividing the largest value of an absolute change rate defined as the absolute value of the change rate of said output signal during a reference period defined as a period from the time at which said oxygen concentration control starts to be performed to the time at which said output signal changing with the performance of said oxygen concentration control converges by the difference between the value of said output signal at the time when said absolute change rate becomes largest and a converged value of said output signal with said reference period; and
an abnormality diagnosis unit (20) that is configured to diagnose abnormality in the responsiveness of said exhaust gas sensor (6) on the basis of said responsiveness index.

## Patentansprüche

1. Diagnosevorrichtung für einen Abgassensor, der auf einen Verbrennungsmotor (1) aufgebracht ist, umfassend den Abgassensor (6), um ein Ausgangssignal auszugeben, das die Sauerstoffkonzentration in Abgas darstellt, wobei der Abgassensor (6) ein Messteil (10) aufweist, das eingerichtet ist, um die Sauerstoffkonzentration in dem Abgas zu messen, umfassend ein Paar von Elektroden, das eine abgasseitige Elektrode (12), die dem Abgas ausgesetzt ist, und eine atmosphärenseitige Elektrode (13), die der Atmosphäre ausgesetzt ist, aufweist, und eine poröse Diffusionsrate begrenzende Schicht (14), die es dem Abgas ermöglicht, sich zu der abgasseitigen Elektrode (12) zu bewegen, während sie die Diffusionsrate des Abgases begrenzt, das zu der abgasseitigen Elektrode (12) geliefert wird, und eine Schutzabdeckung (9), die eingerichtet ist, um das Messteil (10) zu umschließen, und mehrere Luftlöcher (9a) aufweist, umfassend:
eine Steuereinheit (20), die eingerichtet ist, um eine Sauerstoffkonzentrationssteuerung durchzuführen, um die Sauerstoffkonzentration in dem Abgas zu ändern;
eine Berechnungseinheit (20), die eingerichtet ist, um einen Reaktionsfähigkeitsindex zu berechnen, der mit der Reaktionsfähigkeit des Abgassensors (6) korreliert, durch Teilen des größten Werts einer absoluten Änderungsrate, welcher als der absolute Wert der Änderungsrate des Ausgangssignals während eines Referenzzeitraums definiert ist, welcher als ein Zeitraum von dem Zeitpunkt, in dem die Sauerstoffkonzentrationssteuerung beginnt durchgeführt zu werden, bis zu dem Zeitpunkt, in dem das Ausgangssignal, das sich bei der Durchführung der Sauerstoffkonzentrationssteuerung ändert, konvergiert, durch die Differenz zwischen dem Wert des Ausgangssignals zu dem Zeitpunkt, wenn die absolute Änderungsrate am größten wird, und einem konvergierten Wert des Ausgangssignals mit dem Referenzzeitraum; und
eine Reaktionsfähigkeit-Diagnoseeinheit (20), die eingerichtet ist, um die Reaktionsfähigkeit des Abgassensors (6) auf der Basis des Reaktionsfähigkeitsindexes zu schätzen.

2. Diagnosevorrichtung für einen Abgassensor, der auf einen Verbrennungsmotor (1) aufgebracht ist, umfassend den Abgassensor (6), um ein Ausgangssignal auszugeben, das die Sauerstoffkonzentration in Abgas darstellt, wobei der Abgassensor (6) ein Messteil (10) aufweist, das eingerichtet ist, um die Sauerstoffkonzentration in dem Abgas zu messen, umfassend ein Paar von Elektroden, das eine abgasseitige Elektrode (12), die dem Abgas ausgesetzt ist, und eine atmosphärenseitige Elektrode (13), die der Atmosphäre ausgesetzt ist, aufweist, und eine poröse Diffusionsrate begrenzende Schicht (14), die es dem Abgas ermöglicht, sich zu der abgasseitigen Elektrode (12) zu bewegen, während sie die Diffusionsrate des Abgases begrenzt, das zu der abgasseitigen Elektrode (12) geliefert wird, und eine Schutzabdeckung (9), die eingerichtet ist, um das Messteil (10) zu umschließen, und mehrere Luftlöcher (9a) aufweist, umfassend:
eine Steuereinheit (20), die eingerichtet ist, um eine Sauerstoffkonzentrationssteuerung durchzuführen, um die Sauerstoffkonzentration in dem Abgas zu ändern;
eine Berechnungseinheit (20), die eingerichtet ist, um einen Reaktionsfähigkeitsindex zu berechnen, der mit der Reaktionsfähigkeit des Abgassensors (6) korreliert, durch Teilen des größten Werts einer absoluten Änderungsrate, welcher als der absolute Wert der Änderungsrate des Ausgangssignals während eines Referenzzeitraums definiert ist, welcher als ein Zeitraum von dem Zeitpunkt, in dem die Sauerstoffkonzentrationssteuerung beginnt durchgeführt zu werden, bis zu dem Zeitpunkt, in dem das Ausgangssignal, das sich bei der Durchführung der Sauerstoffkonzentrationssteuerung ändert, konvergiert, durch die Differenz zwischen dem Wert des Ausgangssignals zu dem Zeitpunkt, wenn die absolute Änderungsrate am größten wird, und einem konvergierten Wert des Ausgangssignals mit dem Referenzzeitraum; und
eine Anomalie-Diagnoseeinheit (20), die eingerichtet ist, um eine Anomalie in der Reaktionsfähigkeit des Abgassensors (6) auf der Basis des Reaktionsfähigkeitsindexes zu diagnostizieren.

## Revendications

1. Appareil de diagnostic pour un capteur de gaz d'échappement appliqué à un moteur à combustion interne (1) comprenant le capteur de gaz d'échappement (6) pour produire un signal de sortie représentant la concentration en oxygène dans du gaz d'échappement, le capteur de gaz d'échappement (6) ayant une partie de mesure (10) qui est configurée pour mesurer la concentration en oxygène dans le gaz d'échappement comprenant une paire d'électrodes incluant une électrode du côté gaz d'échappement (12) exposée au gaz d'échappement et une électrode du côté atmosphère (13) exposée à l'atmosphère et une couche poreuse de limitation de la vitesse de diffusion (14) qui permet au gaz d'échappement de se déplacer vers ladite électrode du côté gaz d'échappement (12) tout en limitant la vitesse de diffusion du gaz d'échappement délivré vers ladite électrode du côté gaz d'échappement (12), et un capot protecteur (9) qui est configuré pour abriter ladite partie de mesure (10) et incluant une pluralité de trous d'air (9a), comprenant :
une unité de contrôle (20) qui est configurée pour réaliser un contrôle de la concentration en oxygène afin de changer la concentration en oxygène dans le gaz d'échappement ;
une unité de calcul (20) qui est configurée pour calculer un indice de réactivité en corrélation avec la réactivité dudit capteur de gaz d'échappement (6) en divisant la valeur la plus grande d'une vitesse de changement absolue définie comme étant la valeur absolue de la vitesse de changement dudit signal de sortie durant une période de référence définie comme étant une période à compter du moment auquel ledit contrôle de concentration en oxygène commence à être réalisée jusqu'au moment auquel ledit signal de sortie changeant avec la performance dudit contrôle de concentration en oxygène converge par la différence entre la valeur dudit signal de sortie au moment où ladite vitesse de changement absolue devient la plus grande et une valeur convergée dudit signal de sortie avec ladite période de référence ; et
une unité de diagnostic de réactivité (20) qui est configurée pour estimer la réactivité dudit capteur de gaz d'échappement (6) sur la base dudit indice de réactivité.

2. Appareil de diagnostic pour un capteur de gaz d'échappement appliqué à un moteur à combustion interne (1) comprenant le capteur de gaz d'échappement (6) pour produire un signal de sortie représentant la concentration en oxygène dans du gaz d'échappement, le capteur de gaz d'échappement (6) ayant une partie de mesure (10) qui est configurée pour mesurer la concentration en oxygène dans le gaz d'échappement comprenant une paire d'électrodes incluant une électrode du côté gaz d'échappement (12) exposée au gaz d'échappement et une électrode du côté atmosphère (13) exposée à l'atmosphère et une couche poreuse de limitation de la vitesse de diffusion (14) qui permet au gaz d'échappement de se déplacer vers ladite électrode du côté gaz d'échappement (12) tout en limitant la vitesse de diffusion du gaz d'échappement délivré vers ladite électrode du côté gaz d'échappement (12), et un capot protecteur (9) qui est configuré pour abriter ladite partie de mesure (10) et incluant une pluralité de trous d'air (9a), comprenant :
une unité de contrôle (20) qui est configurée pour réaliser un contrôle de la concentration en oxygène afin de changer la concentration en oxygène dans le gaz d'échappement ;
une unité de calcul (20) qui est configurée pour calculer un indice de réactivité en corrélation avec la réactivité dudit capteur de gaz d'échappement (6) en divisant la valeur la plus grande d'une vitesse de changement absolue définie comme étant la valeur absolue de la vitesse de changement dudit signal de sortie durant une période de référence définie comme étant une période à compter du moment auquel ledit contrôle de concentration en oxygène commence à être réalisée jusqu'au moment auquel ledit signal de sortie changeant avec la performance dudit contrôle de concentration en oxygène converge par la différence entre la valeur dudit signal de sortie au moment où ladite vitesse de changement absolue devient la plus grande et une valeur convergée dudit signal de sortie avec ladite période de référence ; et
une unité de diagnostic d'anormalité (20) qui est configurée pour diagnostiquer une anormalité dans la réactivité dudit capteur de gaz d'échappement (6) sur la base dudit indice de réactivité.
